# EUROPÄISCHE PATENTSCHRIFT

(11) **EP 0 986 364 B1**
(45) Veröffentlichungstag und Bekanntmachung des Hinweises auf die Patenterteilung: **14.05.2003**
(21) Anmeldenummer: 98941351.3
(22) Anmeldetag: 16.07.1998
(51) Int. Cl.: A61K 7/00

(54) **MULTIPLE W/O/W-EMULSIONEN MIT HOHEM POLYOLGEHALT**
MULTIPLE WATER/OIL/WATER EMULSIONS WITH A HIGH POLYOL CONTENT
EMULSIONS EAU DANS L'HUILE DANS L'EAU MULTIPLES A FORTE TENEUR EN POLYOLS

(30) Priorität: 25.07.1997 DE 19732013
(43) Veröffentlichungstag der Anmeldung: 22.03.2000
(73) Patentinhaber: Cognis Deutschland GmbH & Co. KG, 40589 Düsseldorf (DE)
(72) Erfinder: TOMINE, Laurence, Robbe, F-77330 Ozoir-la-Ferrière (FR); LE HEN FERRENBACH, Catherine, F-77100 Meaux (FR)
(86) Internationale Anmeldenummer: EP9804453
(87) Internationale Veröffentlichungsnummer: WO99004749

(56) Entgegenhaltungen:
- WO-A1-94/22414
- WO-A1-96/25137
- WO-A1-96/28245
- DE-A1- 4 343 833

## Beschreibung

### Gebiet der Erfindung

Die Erfindung betrifft multiple W/O/W-Emulsionen mit hohem Polyolgehalt, ein Verfahren zu ihrer Herstellung, bei dem man definierte Mengen Polyole in die innere und äußere Phase der Emulsion einarbeitet sowie die Verwendung der Emulsionen zur Herstellung von kosmetischen und/oder pharmazeutischen Zubereitungen.

### Stand der Technik

Multiple Emulsionen stellen Emulsionen von Emulsionen dar. Je nach Herstellung unterscheidet man multiple Wasser/Öl/Wasser (W/O/W)- sowie Öl/Wasser/Öl-(O/W/O)-Emulsionen. Die wichtigste Anwendung multipler Emulsionen besteht darin, Wirkstoffe, die ansonsten nicht miteinander mischbar bzw. konfektionierbar sind, in einer Rezeptur zu verarbeiten. Ein weiterer Vorteil besteht darin, daß die Wirkstoffe kontrolliert über einen längeren Zeitraum freigesetzt werden können. Multiple Emulsionen sind daher insbesondere für die Herstellung von kosmetischen und pharmazeutischen Produkten von besonderer Bedeutung [**Cosm.Toil.105, 65 (1990)].**

Ein besonders elegantes Verfahren zur Herstellung von multiplen Emulsionen wird von S.Matsumoto in **J.Coll.Interf.Sci. 57, 353 (1976)** beschrieben: Hiernach wird zunächst bei erhöhter Temperatur und unter starker Scherung eine Prae-Emulsion hergestellt, die anschließend bei Umgebungstemperatur und unter schwacher Scherung in die wäßrige Lösung eines hydrophilen Emulgators eingebracht wird. Als Emulgatorpaar wird Sorbitanmonoleat und Polyethylenglycol-Derivat eingesetzt. Aus dem umfangreichen Stand der Technik ist femer bekannt, daß als hydrophile Emulgatoren für die Herstellung multipler Emulsionen grundsätzlich Monoglyceride, Sorbitanester, Polysorbate und hochethoxylierte Fettalkohole in Betracht kommen. Stellvertretend sei hier auf die Veröffentlichungen in **Pharm. Acta.Helv. 66, 343 (1991),** sowie von Seiller und Luca in **Bull.Tech./Gattefosse Rep. 80, 27 (1987), S.T.P. Pharma 4, 679 (1988)** und **Int.J.Cosmet.Sci. 13,** 1 (1991) verwiesen. Aus **Yakugaku Zasshi 112, 73 (1992)** sind ferner W/O/W-Emulsionen bekannt, die Glycerintrifettsäureester als Ölkörper und hydrophile Polymere, wie beispielsweise Gelatine, als Stabilisierungsmittel enthalten. Die Verwendung von Albumin und Polyacrylaten als Stabilisatoren für die Wasserphase sowie Niotensiden für die Ölphase ist aus **J.Control.Rel.** 3, 279 (1986) bekannt. Aus der Deutschen Patentschrift **DE-C1 4311445** (Henkel) ist ferner ein Verfahren zur Herstellung multipler W/O/W-Emulsionen bekannt, bei dem man zunächst unter starker Scherung eine W/O-Prae-Emulsion aus Wasser, einem Ölkörper und einem Emulgator I herstellt und diese dann unter schwacher Scherung mit einem wäßrigen Emulgator II behandelt. Als Emulgator I werden dabei entweder Fettsäurepartialglyceride oder Polyglycerinester eingesetzt.

Ein besonderes Problem bei der Herstellung von multiplen W/O/W-Emulsionen besteht jedoch darin, polare Stoffe, insbesondere Polyole wie beispielsweise Glycerin in größeren Mengen (d.h. 10 Gew.-% und mehr) einzuarbeiten, da dies in aller Regel zu klebrigen Emulsionen führt, die vom Verbraucher aus ästhetischen Gründen nicht akzeptiert werden, obschon die sensorische Beurteilung durchaus positiv sein kann.

Die Aufgabe der Erfindung hat somit darin bestanden, ein multiple W/O/W-Emulsionen zur Verfügung zu stellen, die größere Mengen Polyole enthalten. Die Emulsionen sollten dabei elegant, sensorisch einwandfrei und auch bei Temperaturlagerung stabil sein.

### Beschreibung der Erfindung

Gegenstand der Erfindung sind multiple W/O/W-Emulsionen mit hohem Polyolgehalt, die man erhält, indem man zunächst eine Mischung, enthaltend Ölkörper, lipophile Emulgatoren und Polyole zu einer W/O-Prae-Emulsion verarbeitet und diese anschließend mit einer wäßrigen Mischung von hydrophilen Emulgatoren und Polyolen weiterbehandelt, mit der Maßgabe, daß der Gehalt der Polyole in der inneren Phase, also der Prae-Emulsion, 10 bis 50 Gew.-% und in der äußeren Phase 5 bis 20 Gew.-% beträgt.

Überraschenderweise wurde gefunden, daß bei Verteilung der Polyolmengen in den angegebenen Mengen auf die innere und äußere Phase multiple Emulsionen erhalten werden, die das gewünschte Anforderungsprofil erfüllen. Insbesondere das Problem der Klebrigkeit wird zuverlässig vermieden, die Produkte sind vielmehr elegant, d.h. geschmeidig und phasenstabil und spreiten sehr rasch auf der Haut. Die Erfindung schließt die Erkenntnis ein, daß für das verbesserte Erscheinungsbild der Emulsionen und die besondere Stabilität ein Gleichgewicht des osmotischen Druckes zwischen innerer und äußerer Phase entscheidend ist. Wird beispielsweise der osmotische Druck durch Einbringen einer zu großen Menge des Polyols in die innere Phase zu hoch, kann die gewünschte Phaseninversion nicht mehr stattfinden. Wird hingegen die entsprechende Menge Polyol nur in die externe Phase eingebracht, beobachtet man den unerwünschten Effekt der Verklebung. Die Erfindung bietet an Hand der vorgegebenen Mengenangaben eine konkrete Lehre zum technischen Handeln. Der Fachmann kann diese Lehre indes auch dahingehend nutzen, indem er den osmotischen Druck in den beiden Phasen in Abhängigkeit der Einsatzstoffe nach bekannten mathematischen Verfahren berechnet oder mißt und so zu der erforderlichen Verteilung der Polyole in den beiden Phasen kommt. Ein zufriedenstellendes Ergebnis wird stets dann erzielt werden, wenn der osmotische Druck zwischen den beiden Phasen ausgeglichen ist bzw. wenn die Unterschiede minimal sind.

Ein weiterer Gegenstand der Erfindung betrifft ein Verfahren zur Herstellung multipler W/O/W-Emulsionen mit hohem Polyolgehalt, bei dem man man zunächst eine Mischung. enthaltend Ölkörper, lipophile Emulgatoren und Polyole zu einer W/O-Prae-Emuision verarbeitet und diese anschließend mit einer wäßrigen Mischung von hydrophilen Emulgatoren und Polyolen weiterbehandelt, wobei man die Polyole in Mengen von 10 bis 50 Gew.-% - bezogen auf die innere Phase - und in Mengen von 5 bis 20 Gew.-% - bezogen auf die äußere Phase - einsetzt.

### Ölkörper

Als Ölkörper können beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe sowie Siliconöle in Betracht. Die Ölkörper können in Mengen von 10 bis 30, vorzugsweise 15 bis 25 Gew.-% - bezogen auf die Prae-Emulsion - eingesetzt werden.

### Lipophile Emulgatoren

Als Emulgator I kommen Fettsäurepartialester, Polyglycerinester sowie deren Mischungen im Gewichtsverhältnis 96 : 10 bis 10 : 90, vorzugsweise 60 : 40 bis 40 : 60 in Betracht. Typische Beispiele stellen technische Mono- und/oder Diester von Glycerin mit Fettsäuren mit 12 bis 22 Kohlenstoffatomen, wie beispielsweise Glycerinmonolaurat, Glycerinmonopalmitat, Glycerinmonostearat, Glycerinmonoisostearat, Glycerinmonooleat und Glycerinmonobehenat dar. Weitere typische Beispiele sind Mono- und/oder Diester von Oligo- bzw. Polyglyceringemischen (Eigenkondensationsgrad 2 bis 20, vorzugsweise 2 bis 10) der genannten Fettsäuren mit 12 bis 22 Kohlenstoffatomen, wie etwa Polyglycerin-di-isostearat oder Polyglycerin-di-oleat. Als besonders vorteilhaft hat es sich erwiesen, Mischungen von Glycerin- und Oligo- bzw. Polyglycerinestern, beispielsweise bestehend aus Glycerinmonooleat und Triglycerindi-isostearat (Mischungsverhältnis beispielsweise 80 : 20 Gewichtsteile) einzusetzen. Ebenfalls geeignet sind Polyglycerinpolyhydroxystearate, vorzugsweise Polyglycerinpoly-12-hydroxysfearate, wie sie in der Deutschen Patentanmeldung **DE-A1 4420516** (Henkel) beschrieben werden. Diese Stoffe können ihrerseits wieder alleine oder aber in Abmischung mit Zuckertensiden, vorzugsweise Alkyloligoglucosiden oder Fettsäure-N-methylglucamiden eingesetzt werden. Das Gewichtsverhältnis der Partialglyceride und der Polyglycerinester kann 75 : 25 bis 25 : 75 und vorzugsweise 60 : 40 bis 40 : 60 betragen. Die Emulgatoren können in Mengen von 1 bis 10, vorzugsweise 2 bis 8 Gew.-% - bezogen auf die Prae-Emulsion - eingesetzt werden.

### Polyole

Polyole, die im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Der Gehalt der Polyole in der inneren Phase, also der W/O-Prae-Emulsion, kann 10 bis 50, vorzugsweise 20 bis 40 Gew.-% und in der äußeren Phase 5 bis 20, vorzugsweise 7 bis 12 Gew.-% betragen. Vorzugsweise werden zur Herstellung der inneren und äußeren Phase der multiplen Emulsion die gleichen Polyole, insbesondere Glycerin, eingesetzt.

### Wirkstoffe

Im Sinne des erfindungsgemäßen Verfahrens können in die Prae-Emulsion auch Wirkstoffe eingearbeitet werden, deren Stabilisierung sonst üblicherweise Probleme bereitet. Hierzu zählen Siliconöle, Parfümöle, Hydroxycarbonsäuren, Lichtschutzfaktoren, Dihydroxyaceton, Feuchthaltemittel, Depigmentierungsmittel, Antiagingmittel, mikronisierter Titandioxide und/oder Liposomen. Beispiele für **Siliconöle** sind Dimethylpolysiloxane, Methylphenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Die Auswahl der **Parfümöle** ist nicht kritisch; exemplarisch seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle seien genannt Ambroxan, Eugenol, Isoeugenol, Citronellal, Hydroxycitronellal, Geraniol, Citronellol, Geranylacetat, Citral, lonon und Methylionon. Als **Hydroxycarbonsäuren** bzw. deren Salze kommen die bekannten AHA-Säuren in Frage, wie sie beispielsweise in **Ki-Magazin 8, 7 (1995)** beschrieben werden. Unter Lichtschutzfaktoren sind Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfon-säure, 1-(4-tert.Butylphenyl)-3-(4'-methoxyphenyl)-propan-1,3-dion, 3-(4'-Methyl)benzylidenboman-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxiddismutase, Tocopherole (Vitamin E) und Ascorbinsäure (Vitamin C). Beispiele für geeignete **Feuchthaltemittel** sind Hyaluronsäure, Aloe Vera sowie die bereits genannten AHA-Säuren. Als **Depigmen** **tierungsmittel** ("Whitening agents") kommen Hydrochinon, Mulbergextrakt (Maurus Alba), Kojisäure, Mumosa Tenniflora oder Licouce Extrakt in Frage. Als Antiageiningmittel können wiederum Superoxiddismutase, Ginseng-Extrakt und Centella Asiatica eingesetzt werden. Die genannten Wirkstoffe können direkt oder ober ihrerseits schon in liposomaler Form in die Prae-Emulsion eingebracht werden. Ihr Anteil liegt typischerweise bei 1 bis 30, vorzugsweise 2 bis 15 Gew.-% - bezogen auf die Emulsion.

### Hydrophile Emulgatoren

Als hydrophile Emulgatoren kommen Anlagerungsprodukte von durchschnittlich 20 bis 50, vorzugsweise 20 bis 30 Mol Ethylenoxid an Fettalkohole mit 16 bis 22, vorzugsweise 16 bis 18 Kohlenstoffatomen in Betracht. Typische Beispiele sind Addukte von durchschnittlich 15 bis 20 Mol Ethylenoxid an Stearylalkohol, Iso-stearylalkohol, Oleylalkohol, Elaidylalkohol, Petroselinylalkohol, Linolylalkohol, Linolenylalkohol, Ara-chylalkohol, Gadoleylalkohol, Behenylalkohol und Erucylalkohol sowie deren technische Gemische, wie man sie beispielsweise bei der Hochdruckhydrierung von nativen Fettsäuremethylestem oder Aldehyden aus der Roelen'schen Oxosynthese erhält. Vorzugsweise werden Anlagerungsprodukte von durchschnittlich 15 bis 30 Mol Ethylenoxid an technische Cetylstearylalkohole eingesetzt. Als weitere Emulgatoren kommen femer auch Anlagerungsprodukte von durchschnittlich 10 bis 40 Mol Ethylenoxid an Sterole pflanzlicher und/oder tierischer Herkunft in Betracht. Unter dem Begriff Sterole sind hierbei Steroide mit 27 bis 30 Kohlenstoffatomen zu verstehen, die nur am C-3 eine Hydroxygruppe, sonst aber keine funktionellen Gruppen tragen und häufig fälschlich auch als Sterine bezeichnet werden **[ROEMPP Chemie Lexikon, Bd.5, 1992, S.4302].** Typische Beispiele sind Anlagerungsprodukte von durchschnittlich 10 bis 40, vorzugsweise 15 bis 20 Mol Ethylenoxid an Zoosterine, wie etwa Cholesterin, Lanosterin, Spongosterin oder Stellasterin oder Phytosterine, wie etwa Ergosterin, Stigmasterin und Sitosterin. Besonders bevorzugt sind Addukte von durchschnittlich 15 bis 20 Mol Ethylenoxid an Sojasterol.

In einer bevorzugten Ausführungsform der Erfindung werden Mischungen von Anlagerungsprodukten von durchschnittlich 10 bis 50 Mol Ethylenoxid an Fettalkohole mit 16 bis 22 Kohlenstoffatomen und Sterole pflanzlicher und/oder tierischer Herkunft eingesetzt. Ein typisches Beispiel stellt eine Mischung eines Anlagerungsproduktes von durchschnittlich 30 Mol Ethylenoxid an Cetylstearylalkohol und eines Anlagerungsproduktes von durchschnittlich 16 Mol Ethylenoxid an Sojasterol im Gewichtsverhältnis 1 : 5 bis 5 : 1, vorzugsweise 2 : 1 dar. Die hydrophilen Emulgatoren können in Mengen von 1 bis 10, vorzugs-weise 2 bis 8 und insbesondere 2,3 bis 6,5 Gew.-% - bezogen auf die multiple W/O/W-Emulsion - eingesetzt werden. In einer besonderen Ausführungsform der Erfindung können ihnen ferner Fettalkohole mit 12 bis 22, vorzugsweise 16 bis 18 Kohlenstoffatomen als Co-Emulgatoren zugesetzt werden, die in der Emulgatorphase ein flüssigkristallines Netzwerk ausbilden und zu einer weiteren Verbesserung der Stabilität der resultierenden W/O/W-Emulsionen beitragen. Geeignete Fettalkohole sind beispielsweise technische Cetylstearylalkohole. Das Gewichtsverhältnis zwischen hydrophilen Emulgatoren und Co-Emulgator kann 1 : 1 bis 1 : 2, vorzugsweise 1 : 1,5 bis 1 : 1,8 betragen.

### Herstellung der Prae-Emulsion

Zur Herstellung der Prae-Emulsion werden Ölkörper, Polyole und gegebenenfalls die gewünschten Wirkstoffe in einer Rührvorrichtung vorgelegt und mit dem lipophilen Emulgator versetzt. Die Komponenten werden vorzugsweise unter starker Scherung, d. h. bei einer Rührerdrehzahl von 500 bis 2000, vorzugsweise 1200 bis 1700 Upm homogenisiert. Als Rührvorrichtungen kommen beispielsweise Zentripetalturbinen, Kolloidmühlen oder insbesondere Bauteile vom Ultraturrax-Typ in Betracht. Als besonders vorteilhaft hat es sich erwiesen, die Herstellung der Prae-Emulsion bei Temperaturen von 40 bis 90, vorzugsweise 45 bis 80°C durchzuführen. Die Homogenisierzeit liegt üblicherweise im Bereich von 5 bis 40, vorzugsweise 10 bis 30 min. Zur Stabilisierung empfiehlt es sich ferner, der Prae-Emulsion Salz, vorzugsweise Natriumchlorid oder Magnesiumsulfat in Mengen von 0,1 bis 2 Gew.-% - bezogen auf die Prae-Emulsion - zuzusetzen.

Die Zusammensetzung der Prae-Emulsion beträgt somit typischerweise:
(a1) 10 bis 30, vorzugsweise 15 bis 25 Gew.-% Ölkörper,
(a2) 10 bis 50, vorzugsweise 20 bis 40 Gew.-% Polyole,
(a3) 1 bis 10, vorzugsweise 2 bis 8 Gew.-% Emulgator,
(a4) 0 bis 30, vorzugsweise 2 bis 15 Gew.-% Wirkstoffe und
(a5) 0,5 bis 2, vorzugsweise 0,5 bis 1 Gew.-% Salz,
mit der Maßgabe, daß sich die Mengenangaben mit Wasser zu 100 Gew.-% ergänzen. Üblicherweise beträgt der Wassergehalt der Prae-Emulsion 30 bis 60, vorzugsweise 35 bis 55 Gew.-%.

### Herstellung der multiplen W/O/W-Emulsion

Zur Herstellung der multiplen W/O/W-Emulsion wird die Prae-Emulsion in einer Rührvorrichtung vorgelegt und mit einer Mischung, enthaltend den wäßrigen hydrophilen Emulgator und weitere Mengen des Polyols versetzt Die Prae-Emulsion kann dabei in Mengen von 50 bis 90, vorzugsweise 60 bis 80 Gew.-% - bezogen auf die multiple W/O/W-Emulsion - eingesetzt werden. Die Komponenten werden in der Regel unter schwacher Scherung, d. h. bei einer Rührerdrehzahl von 100 bis 1000, vorzugsweise 400 bis 600 Upm homogenisiert. Als Rührvorrichtungen kommen wiederum Zentripetalturbinen oder insbesondere Kolloidmühlen in Betracht. Als besonders vorteilhaft hat es sich erwiesen, die Herstellung der Prae-Emulsion bei 20 bis 60 und insbesondere 20 bis 25°C durchzuführen. Die Homogenisierzeit liegt üblicherweise im Bereich von 5 bis 50, vorzugsweise 10 bis 30 min. Vorzugsweise erfolgt die Herstellung der multiplen W/O/W-Emulsion nach dem Heiß-Kalt-Verfahren, d.h. die Prae-Emulsion wird bei 20 bis 25°C vorgelegt und die auf 40 bis 80°C erhitzte hydrophile Emulgatorphase eingerührt. Wird die Emulgatorphase kalt, also beispielsweise im Bereich von 20 bis 30°C eingerührt, empfiehlt es sich, sie durch Zugabe von Verdickungsmitteln wie beispielsweise Xanthan Gum, Carbopol-Typen und dergleichen zu stabilisieren. Die Zusammensetzung der multiplen W/O/W-Emulsion beträgt somit typischerweise:
(b1) 50 bis 90, vorzugsweise 60 bis 80 Gew.-% Prae-Emulsion,
(b2) 5 bis 20, vorzugsweise 7 bis 12 Gew.-% Polyol,
(b3) 1 bis 10, vorzugsweise 2 bis 8 Gew.-% hydrophiler Emulgator und
(b4) 0 bis 5, vorzugsweise 1 bis 4 Gew.-% Co-Emulgator,
wieder mit der Maßgabe, daß sich die Mengenangaben mit Wasser zu 100 Gew.-% ergänzen. Üblicherweise beträgt der Wassergehalt der multiplen W/O/W- Emulsion - den Wassergehalt der Prae-Emulsion eingerechnet - 45 bis 95, vorzugsweise 50 bis 75 Gew.-%. Der Gesamtgehalt an Polyol - also die Polyolmenge aus der Prae-Emulsion und die Polyolmenge, die bei der Herstellung der externen Phase zugesetzt wird, liegt typischerweise in der Größenordnung von 10 bis 60 und insbesondere 20 bis 50 Gew.-% - bezogen auf die Endformulierung, also die multiple W/O/W-Emulsion.

### Gewerbliche Anwendbarkeit

Die nach dem erfindungsgemäßen Verfahren erhältlichen multiplen W/O/W-Emulsionen können auch unter Einsatz hochpolarer Öle hergestellt werden, erweisen sich auch bei längerer Lagerung stabil und sind leicht biologisch abbaubar. Sie eignen sich zur Aufnahme von größeren Mengen, beispielsweise in Summe von 10 bis 50, vorzugsweise 15 bis 40 und insbesondere 20 bis 30 Gew.-% Polyolen, unter denen Glycerin die bevorzugte Komponente darstellt. Ein weiterer Gegenstand der Erfindung betrifft daher die Verwendung der nach dem erfindungsgemäßen Verfahren erhältlichen multiplen W/O/W-Emulsionen zur Herstellung von kosmetischen und pharmazeutischen Zubereitungen, insbesondere Mitteln zur Haar- und Körperreinigung, und -pflege, in denen die multiplen Emulsionen in Mengen von 1 bis 99, vorzugsweise 10 bis 50 Gew.-% - bezogen auf die Mittel - enthalten sein können

### Beispiele

**Allgemeine Herstellvorschrift.** In einem Ultraturrax wurden Cetiol® S (Dioctylcyclohexan), Dehymuls® B (Polyglyceryl 3-Diisostearate (and) Glyceryl Oleate) sowie Glycerin vorgelegt und mit einer 1 Gew.-%igen wäßrigen Magnesiumsulfatlsöung auf 100 ml aufgefüllt. Danach wurde die Reaktionsmischung bei 80°C und über einen Zeitraum von 10 min bei einer Geschwindigkeit von 1500 Upm homogenisiert. Anschließend wurde die Geschwindigkeit zunächst auf 1125 und dann auf 750 Upm reduziert und jeweils weitere 10 min homogenisiert. 60 g der auf diese Weise hergestellten Prae-Emulsion wurden bei 23°C vorgelegt und innerhalb von 40 s mit einer 70°C heißen wäßrigen'Lösung von Sinnovax® NEVA (PEG-16 Sojasterol (and) Cetearyl Alcohol (and) Ceteareth-20) und weiterem Glycerin versetzt und über einen Zeitraum von 20 min bei einer Geschwindigkeit von 400 Upm homogenisiert. Es resultierten feinteilige W/O/W-Emulsionen, die auch nach 21tägiger Lagerung bei 45°C stabil blieben. Die Zusammensetzungen der Emulsionen ist in Tabelle 1 wiedergegeben.

**Tabelle 1**

| **Multiple W/O/W-Emulsionen (alle Gewichtsangaben als Gew.-%)** | | | | | |
|---|---|---|---|---|---|
| **Zusammensetzung** | **1** | **2** | **3** | **4** | **5** |
| **- Prae-Emulsion** | | | | | |
| Cetiol S | 20,0 | 20,0 | 20,0 | 20,0 | 20,0 |
| Dehymuls B | 4,0 | 4,0 | 4,0 | 4,0 | 4,0 |
| Glycerin | 20,0 | 20,0 | 30,0 | 30,0 | 40,0 |
| Magnesiumsulfat | 0,7 | 0,7 | 0,7 | 0,7 | 0,7 |
| Wasser | ad 100 | | | | |

| ***- Multiple W*/*O*/*W-Emulsion*** | | | | | |
|---|---|---|---|---|---|
| Prae-Emulsion | 60,0 | 60,0 | 60,0 | 60,0 | 60,0 |
| Sinnowax NEVA | 5,6 | 5,6 | 5,6 | 5,6 | 5,6 |
| Glycerin | 10,5 | 5,3 | 15,8 | 7,9 | 10,5 |
| Wasser | ad 100 | | | | |
| ***Gesamt-Glyceringehalt*** | 22,5 | 17,3 | 33,8 | 25,9 | 34,5 |

## Patentansprüche

1. Multiple W/O/W-Emulsionen mit hohem Polyolgehalt, dadurch erhältlich, daß man man zunächst eine Mischung, enthaltend Ölkörper, lipophile Emulgatoren und Polyole zu einer W/O-Prae-Emulsion verarbeitet und diese anschließend mit einer wäßrigen Mischung von hydrophilen Emulgatoren und Polyolen weiterbehandelt, mit der Maßgabe, daß der Gehalt der Polyole in der inneren Phase 10 bis 50 Gew.-%, bezogen auf die innere phase, und in der äußeren Phase 5 bis 20 Gew.-%, bezogen auf die äußere phase beträgt.

2. Verfahren zur Herstellung multipler W/O/W-Emulsionen mit hohem Polyolgehalt, bei dem man zunächst eine Mischung enthaltend Ölkörper, lipophile Emulgatoren und Polyole zu einer W/O-Prae-Emulsion verarbeitet und diese anschließend mit einer wäßrigen Mischung von hydrophilen Emulgatoren und Polyolen weiterbehandelt, **dadurch gekennzeichnet, daß** man die Polyole in Mengen von 10 bis 50 Gew.-% - bezogen auf die innere Phase - und in Mengen von 5 bis 20 Gew.-% - bezogen auf die äußere Phase - einsetzt.

3. Verfahren nach Anspruch 2, **dadurch gekennzeichnet, daß** man Ölkörper einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estem von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C6-C₁₀-Fettsäuren, pflanzlichen Ölen, verzweigten primären Alkoholen, substituierten Cyclohexanen, Guerbetcarbonaten, Dialkylethern und/oder aliphatischen bzw. naphthenischen Kohlenwasserstoffen sowie Siliconölen.

4. Verfahren nach den Ansprüchen 2 und 3, **dadurch gekennzeichnet, daß** man die Ölkörper in Mengen von 10 bis 30 Gew.-% - bezogen auf die Prae-Emulsion - einsetzt.

5. Verfahren nach den Ansprüchen 2 bis 4, **dadurch gekennzeichnet, daß** man lipophile Emulgatoren einsetzt, die ausgewählt sind aus der Gruppe, die gebildet wird von Fettsäurepartialestem, Polyglycerinestem und Polyglycerinpolyhydroxystearaten sowie deren Mischungen.

6. Verfahren nach den Ansprüchen 2 bis 5, **dadurch gekennzeichnet, daß** man die lipophilen Emulgatoren in Mengen von 1 bis 10 Gew.-% - bezogen auf die Prae-Emulsion - einsetzt.

7. Verfahren nach den Ansprüchen 2 bis 6, **dadurch gekennzeichnet, daß** man Polyole einsetzt, die ausgewählt sind aus der Gruppe, die gebildet wird von Glycerin, Alkylenglycolen, technischen Oligoglyceringemischen, Methylolverbindungen, Niedrigalkylglucosiden, Zuckeralkoholen, Zuckern und Aminozuckern.

8. Verfahren nach den Ansprüchen 2 bis 7, **dadurch gekennzeichnet, daß** man bei der Herstellung der W/O-Prae-Emulsion weiterhin Wirkstoffe einsetzt, die ausgewählt sind aus der Gruppe, die gebildet wird von Siliconölen, Parfümölen, Hydroxycarbonsäuren, Lichtschutzfaktoren, Dihydroxyaceton, Feuchthaltemitteln, Depigmentierungsmitteln, Antiagingmitteln, mikronisierten Titandioxiden und/oder Liposomen.

9. Verfahren nach den Ansprüchen 2 bis 8, **dadurch gekennzeichnet, daß** man die Wirkstoffe in Mengen von 1 bis 30 Gew.-% - bezogen auf die Prae-Emulsion - einsetzt.

10. Verfahren nach den Ansprüchen 2 bis 9, **dadurch gekennzeichnet, daß** man hydrophile Emulgatoren einsetzt, die ausgewählt sind aus der Gruppe, die gebildet wird von Anlagerungsprodukten von durchschnittlich 20 bis 50 Mol Ethylenoxid an Fettalkohole mit 16 bis 22 Kohlenstoffatomen und Anlagerungsprodukten von durchschnittlich 10 bis 40 Mol Ethylenoxid an Sterole pflanzlicher und/oder tierischer Herkunft.

11. Verfahren nach den Ansprüchen 2 bis 10, **dadurch gekennzeichnet, daß** man die hydrophilen Emulgatoren in Mengen von 1 bis 10 Gew.-% - bezogen auf die multiple W/O/W- Emulsion - einsetzt.

12. Verwendung der multiplen W/O/W-Emulsionen nach Anspruch 1 zur Herstellung von kosmetischen und/oder pharmazeutischen Zubereitungen.

## Claims

1. Multiple w/o/w emulsions with a high polyol content obtainable by first processing a mixture containing oil components, lipophilic emulsifiers and polyols to form a w/o pre-emulsion and then treating the w/o pre-emulsion thus formed with an aqueous mixture of hydrophilic emulsifiers and polyols, with the proviso that the polyol content in the inner phase is from 10 to 50% by weight, based on the inner phase, and in the outer phase from 5 to 20% by weight, based on the outer phase.

2. A process for the production of multiple w/o/w emulsions with a high polyol content in which a mixture containing oil components, lipophilic emulsifiers and polyols is first processed to form a w/o pre-emulsion and the w/o pre-emulsion thus formed is then treated with an aqueous mixture of hydrophilic emulsifiers and polyols, **characterized in that** the polyols are used in quantities of 10 to 50% by weight, based on the inner phase, and in quantities of 5 to 20% by weight, based on the outer phase.

3. A process as claimed in claim 2, **characterized in that** oil components selected from the group consisting of Guerbet alcohols based on C₆₋₁₈ fatty alcohols, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, vegetable oils, branched primary alcohols, substituted cyclohexanes, Guerbet carbonates, dialkyl ethers and/or aliphatic or naphthenic hydrocarbons and silicone oils are used.

4. A process as claimed in claims 2 and 3, **characterized in that** the oil components are used in quantities of 10 to 30% by weight, based on the pre-emulsion.

5. A process as claimed in claims 2 to 4, **characterized in that** lipophilic emulsifiers selected from the group consisting of fatty acid partial esters, polyglycerol esters and polyglycerol polyhydroxystearates and mixtures thereof are used.

6. A process as claimed in claims 2 to 5, **characterized in that** the lipophilic emulsifiers are used in quantities of 1 to 10% by weight, based on the pre-emulsion.

7. A process as claimed in claims 2 to 6, **characterized in that** polyols selected from the group consisting of glycerol, alkylene glycols, technical oligoglycerol mixtures, methylol compounds, lower alkyl glucosides, sugar alcohols, sugars and amino sugars are used.

8. A process as claimed in claims 2 to 7, **characterized in that** active substances selected from the group consisting of silicone oils, perfume oils, hydroxycarboxylic acids, UV protection factors, dihydroxyacetone, moisturizers, depigmenting agents, antiaging agents, micronized titanium dioxides and/or liposomes are additionally used in the preparation of the pre-emulsion.

9. A process as claimed in claims 2 to 8, **characterized in that** the active substances are used in quantities of 1 to 30% by weight, based on the pre-emulsion.

10. A process as claimed in claims 2 to 9, **characterized in that** hydrophilic emulsifiers selected from the group consisting of products of the addition of on average 20 to 50 mol ethylene oxide onto C₁₆₋₂₂ fatty alcohols and products of the addition of on average 10 to 40 mol ethylene oxide onto sterols of vegetable and/or animal origin are used.

11. A process as claimed in claims 2 to 10, **characterized in that** the hydrophilic emulsifiers are used in quantities of 1 to 10% by weight, based on the multiple w/o/w emulsion.

12. The use of the multiple w/o/w emulsions claimed in claim 1 for the production of cosmetic and/or pharmaceutical preparations.

## Revendications

1. Emulsions Eau/Huile/Eeau W/O/W multiples ayant une teneur élevée en polyol, accessibles en ce qu'on fabrique en premier lieu un mélange contenant des composés huileux, des agents émulsionnants lipophiles, et des polyols en vue d'une pré-émulsion W/O et en ce qu'on poursuit le traitement ensuite de celle-ci avec un mélange aqueux d'agents émulsionnants et de polyols,
avec la précision que la teneur des polyols dans la phase interne s'élève à 10 à 50 % en poids rapporté à la phase interne - et dans la phase externe s'élève à 5 à 20 % en poids rapporté à la phase externe.

2. Procédé de préparation d'émulsions W/O/W ayant une teneur élevée en polyol dans lequel on produit en premier lieu un mélange contenant des composés huileux, des agents émulsionnants lipophiles et des polyols en une pré-émulsion W/O et en ce qu'on poursuit le traitement de celle-ci ensuite avec un mélange aqueux d'agents émulsionnants hydrophiles et de polyols,
**caractérisé en ce qu'**
on met en oeuvre les polyols en des quantités allant de 10 à 50 % en poids - rapporté à la phase interne - et en quantités allant de 5 à 20 % en poids - rapporté à la phase externe.

3. Procédé selon la revendication 2,
**caractérisé en ce qu'**
on met en oeuvre des composés huileux qui sont choisis dans le groupe formé des alcools de Guerbet à base d'alcools gras ayant de 6 à 18 atomes de carbone, des esters d'acides gras en C₆-C₂₀ linéaires avec des alcools gras linéaires en C₆-C₂₀, des esters d'acides carboxyliques en C₆-C₁₃ ramifiés avec des alcools gras en C₆-C₂₀ linéaires, des esters d'acides gras en C₆-C₁₈ linéaires avec des alcools ramifiés, des esters d'acides gras linéaires et/ou ramifiés avec des alcools plurifonctionnels et/ou des alcools de Guerbet, des triglycérides à base d'acides gras en C₆-C₁₀, des huiles végétales, des alcools primaires ramifiés, des cyclohexanes substitués, des carbonates de Guerbet, des éthers dialkyliques, et/ou des hydrocarbures aliphatiques naphténiques ainsi que des huiles de silicone.

4. Procédé selon les revendications 2 et 3,
**caractérisé en ce qu'**
on met en oeuvre les composés huileux en des quantités allant de 10 à 30 % en poids rapporté à la pré-émulsion.

5. Procédé selon les revendications 2 à 4,
**caractérisé en ce qu'**
on met en oeuvre des agents émulsionnants lipophiles qui sont choisis dans le groupe formé des esters partiels d'acide gras, des esters de polyglycérol, et des polyhydroxystéarates de polyglycérol, ainsi que de leurs mélanges.

6. Procédé selon les revendications 2 à 5,
**caractérisé en ce qu'**
on met en oeuvre les agents émulsionnants lipophiles en quantités allant de 1 à 10 % en poids rapporté à la pré-émulsion.

7. Procédé selon les revendications 2 à 6,
**caractérisé en ce qu'**
on met en oeuvre des polyols qui sont choisis dans le groupe formé du glycérol, des alkylène glycols, des mélanges industriels d'oligoglycérols, des composés méthyloliques, des (alkyls inférieurs) glucosides, des alcools de sucre, des sucres, et des aminosucres.

8. Procédé selon les revendications 2 à 7,
**caractérisé en ce qu'**
on met en oeuvre lors de la préparation de la pré-émulsion W/O en outre des principes actifs qui sont choisis dans le groupe formé des huiles de silicone des essences de parfum, des acides hydrocarboxyliques, des facteurs de protection contre les lumières, de la dihydroxyacétone, des agents qui retiennent l'humidité, des agents de dépigmentation, des agents antivieillissement, des dioxydes de titane micronisés et/ou des liposomes.

9. Procédé selon les revendications 2 à 8,
**caractérisé en ce qu'**
on met en oeuvre les principes actifs en des quantités allant de 1 à 30 % en poids - rapporté à la pré-émulsion.

10. Procédé selon les revendications 2 à 9,
**caractérisé en ce qu'**
on met en oeuvre des agents émulsionnants hydrophiles qui sont choisis dans le groupe formé des produits d'addition de -en moyenne - 20 à 50 mol d'oxyde d'éthylène sur des alcools gras ayant de 16 à 22 atomes de carbone et des produits d'addition de - en moyenne - 10 à 40 mol d'oxyde d'éthylène sur des stérols d'origine végétale et/ou animale.

11. Procédé selon les revendications 2 à 10,
**caractérisé en ce qu'**
on met en oeuvre les agents émulsionnants hydrophiles en quantités allant de 1 à 10 % en poids - rapporté à l'émulsion multiple W/O/W.

12. Utilisation des émulsions multiples W/O/W selon la revendication 1, pour la production de préparation cosmétiques et/ou pharmaceutiques.
